# EUROPEAN PATENT APPLICATION

(11) **EP 4 062 883 A1**
(43) Date of publication of application: **28.09.2022**
(21) Application number: 20889215.8
(22) Date of filing: 18.11.2020
(51) Int. Cl.: A61F 13/49, A61F 13/51

(54) **DISPOSABLE DIAPER**

(30) Priority: 20.11.2019 JP 2019209787
(71) Applicant: Daio Paper Corporation, Ehime 799-0492 (JP)
(72) Inventor: OKADA, Yuki, Shikokuchuo-shi, Ehime 799-0431 (JP)
(74) Representative: Held, Stephan
(86) International application number: PCT/JP2020/043055
(87) International publication number: WO 2021/100776

(57) **Abstract**

The problem is to provide a disposable diaper having improved air permeability in its wait region irrespective of the configuration of an elastic member. The problem is solved by a disposable diaper including top sheet 30 constituting a use side surface, liquid-impervious sheet 11 provided on the under face side, and an absorbent element interposed therebetween, wherein cover nonwoven sheet 20 is provided on the under face side of the liquid-impervious sheet 11, the cover nonwoven sheet 20 being formed of perforated nonwoven fabric having a number of apertures arranged at intervals and each penetrating two sides of the fabric, and imaginary lines connecting the adjacent apertures located in a waist region of the cover nonwoven sheet form a Moroccan pattern D, and at least part of the Moroccan pattern D does not overlap an absorber body 56 contained in the absorbent element 50.

## Description

### TECHNICAL FIELD

The present invention relates to disposable diapers.

### BACKGROUND ART

It is a common technique to provide a tape-type as well as an underpants-type disposable diaper with a waist elastic member for the purpose of preventing leakage through waist portion of the diaper.

Baby diapers, in particular, are prone to cause so-called diaper rash as the skin of babies is sensitive.

Above all, the waist region is brought into close contact with the skin by means of the waist elastic member, which tends to cause moisture to stay inside in the absence of an absorber body or a member that absorbs moisture, and is thus prone to cause skin rash due to dampness.

In order to avoid skin rash, it is important to secure air permeability between interior and exterior of a diaper. In this regard, Patent Literature 1 proposes to provide a net-shaped elastic member to improve air permeability.

### PRIOR ART LITERATURE

### PATENT LITERATURE

Patent Literature 1: JP 2012-65849 A

### SUMMARY OF THE INVENTION

### PROBLEMS TO BE SOLVED BY THE INVENTION

Yet, the net-shaped elastic member proposed for use in the prior art is not for general purpose.

Therefore, it is a primary object of the present invention to provide a disposable diaper in which air permeability is improved in the wait region irrespective of the configuration of the elastic member.

### MEANS FOR SOLVING THE PROBLEM

Typical aspects of the present invention solving the above problem are as follows.

### <Typical Aspect>

A disposable diaper including a top sheet constituting a use side surface, a liquid-impervious sheet provided on an under face side, and an absorbent element interposed therebetween,
wherein a cover nonwoven sheet is provided on an under face side of the liquid-impervious sheet, the cover nonwoven sheet being formed of perforated nonwoven fabric having a number of apertures arranged at intervals and each penetrating two sides of the fabric, and
imaginary lines connecting the adjacent apertures located in a waist region of the cover nonwoven sheet form a Moroccan pattern, and at least part of the Moroccan pattern does not overlap an absorber body contained in the absorbent element.

### EFFECT OF THE INVENTION

According to the present invention, advantages such as improved air permeability in the waist region irrespective of the configuration of the elastic member may be achieved.

### BRIEF DESCRIPTION OF THE DRAWINGS

Fig. 1 is a plan view of a tape-type disposable diaper in its spread state, illustrating the inner surface thereof.
Fig. 2 is a plan view of the tape-type disposable diaper in its spread state, illustrating the outer surface thereof.
Fig. 3 is a cross sectional view taken along lines 3-3 in Fig. 1.
Fig. 4 is a cross sectional view taken along lines 4-4 in Fig. 1.
Fig. 5(a) is a cross sectional view taken along lines 5a-5a in Fig. 1, Fig. 5(b) is a cross sectional view taken along lines 5b-5b in Fig. 1, and Fig. 5(c) is a cross sectional view taken along lines 5c-5c in Fig. 1.
Fig. 6 shows enlarged plan views of a relevant part of examples of the aperture pattern in the top sheet or cover nonwoven sheet.
Fig. 7 is an enlarged plan view of a relevant part of an example of the aperture pattern in the top sheet or cover nonwoven sheet.
Fig. 8 is a photograph of a diaper taken from the top sheet side thereof in Comparative Example 1.
Figs. 9(a) and 9(b) are photographs of the diaper taken from the dorsal end side thereof in Comparative Example 1.
Fig. 10 is a photograph of a diaper taken from the top sheet side thereof in Comparative Example 2.
Figs. 11(a) and 11(b) are photographs of the diaper taken from the dorsal end side thereof in Comparative Example 2.
Figs. 12(a) and 12(b) are photographs of a diaper taken from the top sheet side thereof in Example.
Figs. 13(a) and 13(b) are photographs of the diaper taken from the dorsal end side thereof in Example.

### EMBODIMENTS FOR CARRYING OUT THE INVENTION

Embodiments of the present invention will now be explained in detail with reference to the attached drawings. In sectional views, a dotted pattern region represent an adhesive as joining means for joining the components on the top side and the underside of the region, and may be formed by, for example, solid, bead, curtain, summit, or spiral application, or pattern coating (transfer of a hot melt adhesive by relief printing) of a hot melt adhesive, or fixed portions of the elastic members may be formed, in place of or in addition to the above, by application of a hot melt adhesive to the external surface of the elastic members with a comb gun or a surewrap. Examples of the hot melt adhesive include, but not limited to, EVA-based, adherent rubber-based (elastomer-based), olefin-based, and polyester/polyamide-based adhesives. The joining means for joining components may alternatively be material melt-bonding, such as heat sealing or ultrasonic sealing.

### <Example of tape-type disposable diaper>

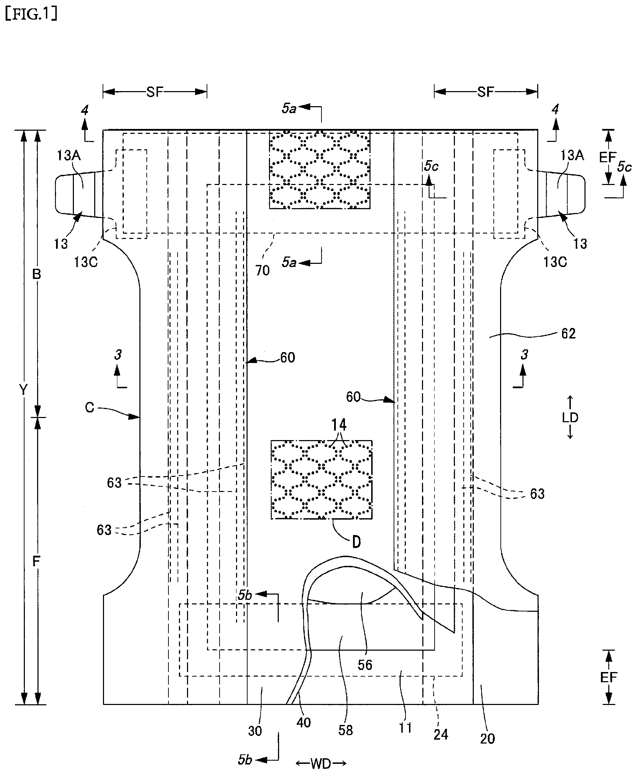

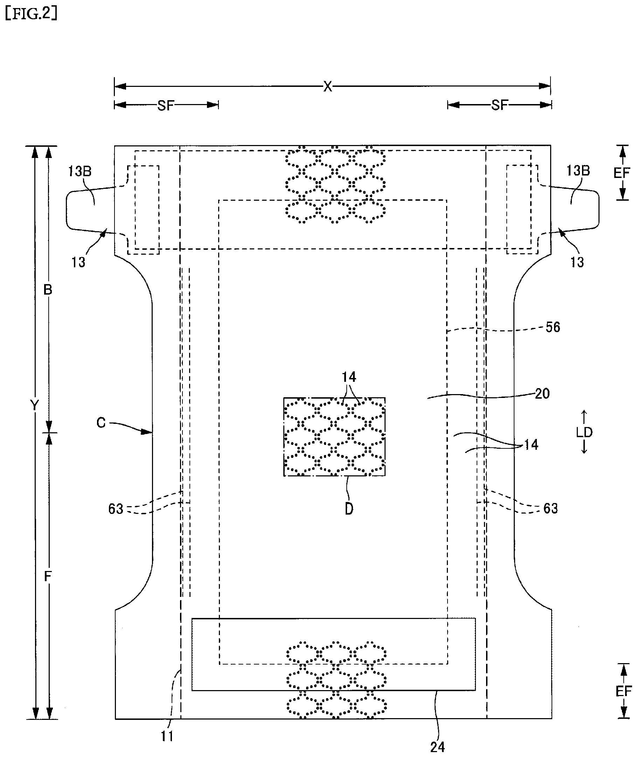

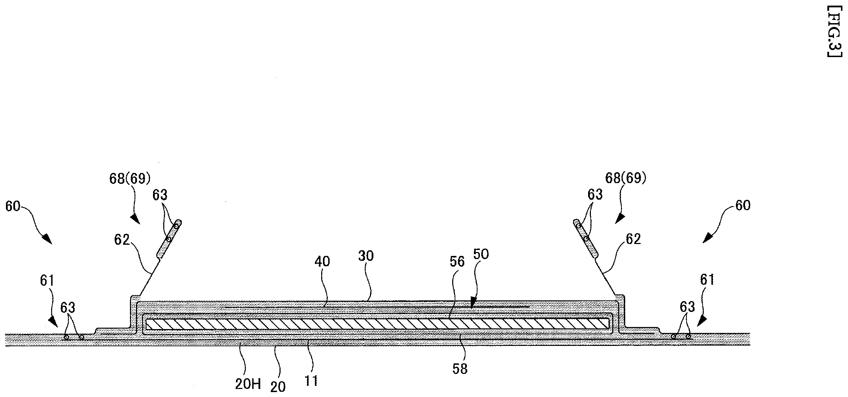

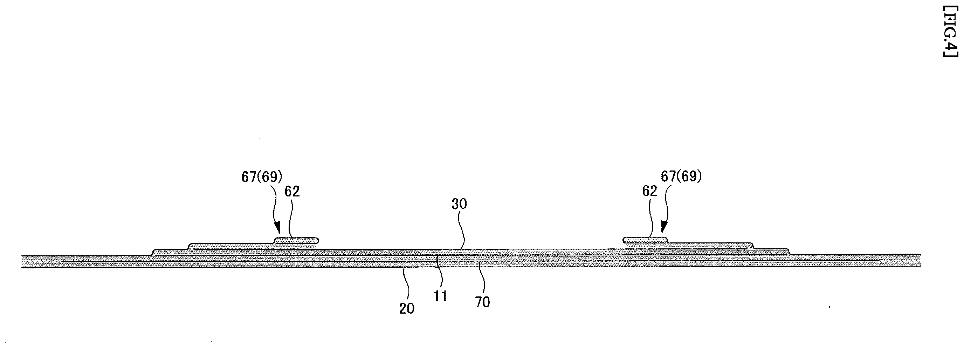

Figs. 1 to 5 show an example of a tape-type disposable diaper as a disposable diaper according to the present invention, wherein X refers to the overall width of the diaper except for the fastening tapes, and Y refers to the overall length of the diaper. This tape-type disposable diaper has an absorber body 56 extending from the ventral to dorsal sides, a liquid-pervious top sheet 30 covering the top side of the absorber body 56, and a liquid-impervious sheet 11 covering the underside of the absorber body 56, and has a ventral end flap EF and a dorsal end flap EF each extending on the front and back sides, respectively, of the absorber body 56 and not containing the absorber body 56, and a pair of side flaps SF extending laterally from the opposed side edges of the absorber body 56. Each side flap SF has a narrowed portion in the middle of the front-back direction, which will fit around each leg, and a fastening tape 13 on the dorsal side of the narrowed portion.

The liquid-impervious sheet 11 is covered on its under face with a cover nonwoven sheet 20. The cover nonwoven sheet 20 extends to the peripheries of the diaper, whereas the liquid-impervious sheet 11 extends in the front-back direction up to the front and back edges of the diaper, and in the width direction up to between each side edge of the absorber body 56 and each side edge of the cover nonwoven sheet 20. However, the cover nonwoven sheet 20 may extend partially in the front-back direction, partially in the width direction, or both, as necessary. For example, when part of the liquid-impervious sheet 11 is covered with another material, such as gather nonwoven fabric, the cover nonwoven sheet 20 may not be provided over that part.

The top sheet 30 and the liquid-impervious sheet 11 in the illustrated embodiment are rectangular, and have dimensions slightly larger in the front-back and width directions than those of the absorbent element 50. The peripheral portions of the top sheet 30 extending beyond the side edges of the absorbent element 50 and the peripheral portions of the liquid-impervious sheet 11 extending beyond the side edges of the absorbent element 50 are joined, for example, with a hot melt adhesive.

The absorbent element 50 includes an absorber body 56 and a packing sheet 58 covering the absorber body 56, and may be interposed between the top sheet 30 and the liquid-impervious sheet 11, while an intermediate sheet 40 may be interposed between the top sheet 30 and the absorbent element 50.

The intermediate sheet 40 in the illustrated embodiment is shorter than the width of the absorbent element 50 and disposed in the center, but may be provided all over the width. The longitudinal dimension of the intermediate sheet 40 may be the same as the overall length of the diaper, the same as the length of the absorbent element 50, or within a small length range around the area where liquids are received. In addition, an indicator may be provided which changes color upon contact with a liquid component of the bodily waste.

On the top face of the tape-type disposable diaper on each side in the width direction, a side gather part 60 is provided. Each side gather part 60 has a first portion 61 provided on the side flap SF (planar gather portion) and a second portion 69 protruding over each side portion of the top sheet 30 (three-dimensional gather portion).

Specifically, a strip of gather nonwoven fabric 62 having the same length as the overall length Y of the diaper extends from the first portion 61 to the second portion 69. In the first portion 61, the gather nonwoven fabric strip 62 is joined to the cover nonwoven sheet 20, for example, with a hot melt adhesive, and between these nonwoven strip and sheet, one or a plurality at intervals in the width direction WD, of gathering elastic members 63 extending in the front-back direction LD are fixed in the extended state and, with the contracting force of the gathering elastic members, the first portion 61 is contracted in the front-back direction LD to form planar gathers, which will be brought into contact around each leg.

The gather nonwoven fabric strip 62 has an extending portion extending from the first portion 61 as the base toward the center of the width direction WD, and at least this extending portion is folded back in the leading edge portion to form a double-layered structure. The ends opposed in the front-back direction LD of the extending portion are fixed to the top sheet 30 to form laid-down portions 67, whereas the middle portion in the front-back direction LD located between the laid-down portions 67 forms a non-fixed, free portion 68. In the free portion 68, one or the plurality at intervals in the width direction WD, of gathering elastic members 63 extending in the front-back direction LD are fixed in the extended state and, with their contracting force, the free portion 68 of the second portion 69 is contracted in the front-back direction LD to form three-dimensional gathers, which will be brought into contact around each leg.

The fastening tape 13 in the illustrated embodiment has a base sheet forming a tape attachment portion 13C fixed to a side portion of the diaper and a tape body 13B protruding from the tape attachment portion 13C, and an engaging part 13A disposed on the tape body 13B of the base sheet in the middle of the width direction and to be engaged on the ventral side, and the portion beyond this engaging part 13A is a grip portion. The tape attachment portion 13C of the fastening tape 13 is interposed between the gather nonwoven fabric 62 as an inner layer and the cover nonwoven sheet 20 as an outer layer of the side flap, and adhered thereto with a hot melt adhesive. The engaging part 13A is joined to the inner surface of the tape body 13B with an adhesive.

The engaging part 13A may preferably be a hook member (male part) of a mechanical fastener (hook and loop fastener). The hook member has a number of engaging projections on its outer surface. The engaging projections may be (A) tick-shaped, (B) J-shaped, (C) mushroom-shaped, (D) T-shaped, (E) double J-shaped (wherein J-shaped parts are joined back to back), or the like, and may be in any of these. Needless to say, the engaging part of the fastening tape 13 may be a sticky material layer.

The base sheet forming from the tape attachment portion 13C to the tape body 13B may be of nonwoven fabric, plastic film, polyethylene-laminated nonwoven fabric, paper, or composites thereof.

For fitting the diaper on a wearer, with the dorsal side flaps SF overlapping the exterior of the ventral side flaps SF, the fastening tapes 13 are engaged in the ventral region F on the outer surface at appropriate sites. The position and the size of the sites to be engaged by the fastening tapes 13 may be decided arbitrarily.

At the sites in the ventral region F to be engaged by the fastening tapes 13, a target sheet 24 having targets for facilitating the engagement is preferably disposed. When the engaging part 13A is a hook member, the target sheet 24 may preferably be a film type having a film layer and an engaging layer provided over the outer surface of the film layer, on which engaging layer the hooks of the engaging part 13A detachably engage. In this case, the engaging layer is known to be a thread-knit web having loops, which is to be attached to the film layer, or a nonwoven layer of a thermoplastic resin, which is to be attached to the film layer through intermittent ultrasonic seals so that the fibers of the nonwoven fabric form loops. Either of these may preferably be used. Further, a filmless target tape may also be used, which is formed of embossed nonwoven fabric of a thermoplastic resin and has no film layer. On such a target tape, fastening tapes 13 engage with the hooks thereof by being entangled or hooked on the loops.

When the engaging part 13A is a sticky material layer, a base sheet made of plastic film with a sticky smooth surface which has been subjected to release lining, may be used.

When the sites in the ventral region F to be engaged by the fastening tapes 13 are of nonwoven fabric, e.g., when the cover nonwoven sheet 20 in the illustrated embodiment is of nonwoven fabric, and the engaging parts 13A of the fastening tapes 13 are hook members, the target sheet 24 may be omitted, and the hook members may be caught on the nonwoven fabric of the cover nonwoven sheet 20 for engagement. In this case, the target sheet 24 may be interposed between the cover nonwoven sheet 20 and the liquid-impervious sheet 11.

The end flaps EF extend on the front and back sides of the absorbent element 50 and do not contain the absorber body 56. Extending on the front side is the ventral end flap EF, and extending on the back side is the dorsal end flap EF.

The front-back dimension of the dorsal end flap EF is preferably the same as or smaller than the front-back dimension of the attachment portion of the fastening tape 13 for the reason mentioned above. If the absorbent element 50 is positioned too close to the dorsal end portion of the diaper, the thickness and stiffness of the absorbent element 50 tend to cause a gap between the dorsal end portion of the diaper and the body surface, so that the front-back dimension of the dorsal end flap EF is preferably 10 mm or larger.

The front-back dimensions of the ventral end flap EF and the dorsal end flap EF are preferably about 5 to 20 % the front-back dimension Y of the overall diaper, and in baby diapers, 10 to 60 mm, particularly 20 to 50 mm.

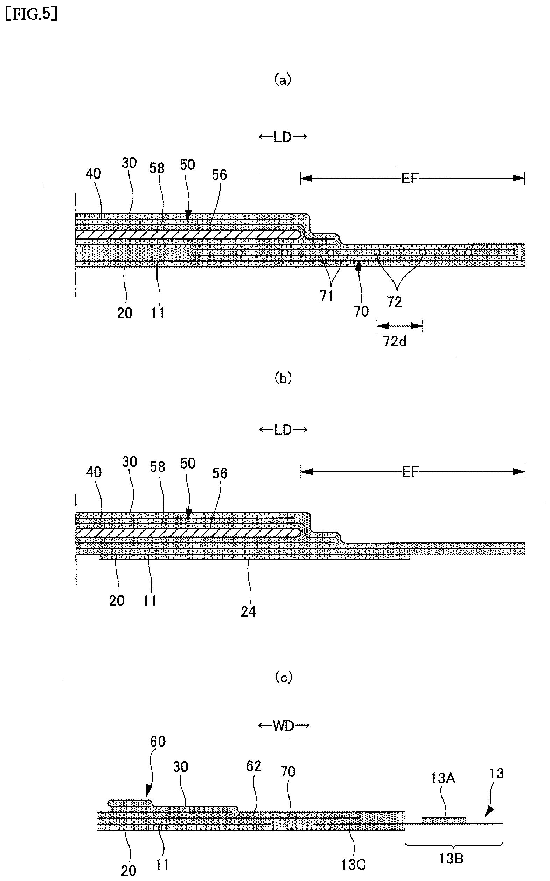

For improved dorsal fitting of the diaper, as shown in detail in Fig. 5, an elastic member which is elastically stretchable in the width direction, in particular, a strip-shaped dorsal stretchable sheet 70 is preferably provided between the opposed fastening tapes 13. Each end portion of the dorsal stretchable sheet 70 preferably extends to overlap the attachment portion of the corresponding fastening tape 13, but may be spaced apart from the attachment portion toward the center of the width direction. The front-back dimension of the dorsal stretchable sheet 70 is preferably about 20 % larger or smaller than the front-back dimension of the attachment portion of the fastening tape 13. Further, when the dorsal stretchable sheet 70 is arranged overlapping the boundary between the dorsal end flap EF and the absorbent element 50 as illustrated, the dorsal end portion of the absorbent element 50 is pressed tightly onto the body, which is preferable.

The dorsal stretchable sheet 70 may be a sheet-shaped elastic member like a rubber sheet but, in view of air permeability, may preferably be nonwoven fabric or paper. In this case, air-permeable sheet-shaped elastic member, such as of stretchable nonwoven fabric may be used but, as shown in Fig. 5(a), it is preferred to use two base sheets 71, such as of nonwoven fabric, bonded together with an adhesive, such as a hot melt adhesive, to fix therebetween elastic members 72 in the form of a perforated sheet, web, or elongate (thread, string, or the like) shape, or the like, in the stretched state in the width direction. The base sheet 71 here may be of a material similar to the cover nonwoven sheet 20. The elastic members 72 preferably have an elongation of about 150 to 250 %. When the elastic members 72 are in an elongate shape (thread, string, or the like), it is preferred to arrange five to fifteen threads of the elastic members each having a fineness of 420 to 1120 dtex, at 3 to 10 mm intervals.

As shown in Fig. 5(a), by arranging part of the elastic members 72 across the absorbent element 50, fitting of the absorbent element 50 is preferably improved. In this case, by causing part or all of the elastic members 72 superimposed on the absorbent element 50 to lose their contracting force, e.g., by cutting, the absorbent element 50 is kept from contracting in the width direction in its dorsal end portion, which further improves fitting.

Note that the elastic members 72 may be fixed over the entire length of the base sheets 71 along the longitudinal direction of the sheet (width direction of the diaper) but, for preventing contraction or turning-over of the sheet upon attachment to the diaper body, the contracting force may be caused to be lost or the elastic members 72 may be caused to be absent in the area of about 5 to 20 mm from each end of the sheet in the front-back direction (width direction of the diaper). In the absence of the elastic members 72, a frill out of contact with the skin may be formed, which increase air permeability.

The dorsal stretchable sheet 70, in the illustrated embodiment, is interposed between the gather nonwoven fabric 62 and the cover nonwoven sheet 20 on each lateral side of the liquid-impervious sheet 11 in the width direction, and between the liquid-impervious sheet 11 and the absorbent element 50 in the area overlapping the liquid-impervious sheet 11, but may be placed between the liquid-impervious sheet 11 and the cover nonwoven sheet 20, on the exterior surface of the cover nonwoven sheet 20, or between the top sheet 30 and the absorbent element 50.

Further, the dorsal stretchable sheet 70 may be placed on the top sheet 30 and, in this case, on the gather nonwoven fabric 62 on each lateral side of the liquid-impervious sheet 11 in the width direction. When the cover nonwoven sheet 20 is formed by stacking a plurality of base sheets, the entire dorsal stretchable sheet 70 may be interposed between the base sheets of such cover nonwoven sheet 20.

### <Basic structure of the present invention>

The disposable diaper according to the present invention has, referring to the explanatory reference numerals used in the above discussion of the embodiment, a top sheet 30 constituting a use side surface, a liquid-impervious sheet 11 provided on the under face side, and an absorbent element 50 interposed therebetween,
wherein a cover nonwoven sheet 20 is provided on the under face side of the liquid-impervious sheet 11, the cover nonwoven sheet 20 being formed of perforated nonwoven fabric having a number of apertures 14 arranged at intervals and each penetrating two sides of the fabric, and
wherein imaginary lines connecting the adjacent apertures 14 located I a waist region of the cover nonwoven sheet 20 form a Moroccan pattern D, and at least part of the Moroccan pattern D does not overlap an absorber body 56 contained in the absorbent element 50.

According to the basic structure of the present invention, when the cover nonwoven sheet 20 is formed of perforated nonwoven fabric having at intervals a number of apertures 14 penetrating two sides of the fabric, the vapor from the absorbent element 50 side passing through ventilation micropores (not shown), which are formed in the liquid-impervious sheet 11 and has moisture permeability to permeate only the vapor component and not to permeate the liquid component, may be easily discharged through the apertures 14 to outside. In the area without the liquid-impervious sheet 11, the cover nonwoven sheet 20 may easily discharge the vapor directly through the apertures 14 to outside. In this way, the dampness may be avoided.

The packing sheet 58 covering the absorber body 56 may be formed of tissue paper, in particular, crepe paper, or SMS nonwoven fabric or SMMS nonwoven fabric may be used. SMS nonwoven fabric and SMMS nonwoven fabric are soft and have a low rigidity.

With SMS nonwoven fabric or SMMS nonwoven fabric, a disposable diaper, when worn, deforms well (bends well) in response to the posture change of the wearer and is securely kept in contact with the skin of the wearer, which leads to improved leak prevention.

Further, when the moisturizer M composed mainly of glycerin is applied to the exterior surface of the top sheet 30 (containing the moisturizer M at least in the exterior surface portion), the moisturizer M performs functions not only to protect the skin of a wearer, but also to reduce friction with the skin of a wearer.

As a result, when the disposable diaper is worn, the top sheet slides with respect to the skin in response to the posture change of a wearer to ensure contact with the skin of the wearer, which improves the leak prevention effect.

The top sheet 30 is preferably formed of perforated nonwoven fabric having a number of apertures 14. Though the exact reason is not known, a number of apertures 14 makes smaller the area in contact with the skin of a wearer compared to nonwoven fabric without apertures, to thereby exhibit function to reduce friction with the skin of the wearer.

As a result, when the disposable diaper is worn, the top sheet slides with respect to the skin in response to the posture change of a wearer to ensure contact with the skin of the wearer, which improves the leak prevention effect.

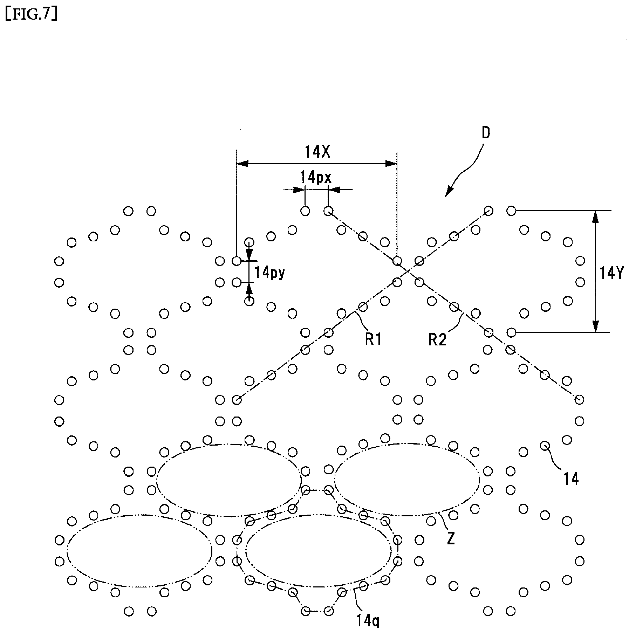

In addition to the apertures 14 in the cover nonwoven sheet 20, the apertures 14 in also the top sheet 30 suitably exhibits the plan arrangement such that the imaginary lines 14q connecting adjacent apertures 14 form a Moroccan pattern D, as typically shown in Fig. 7.

The exact reason why the Moroccan pattern D provides advantages is not clear, but it is assumed to be because the imaginary linking lines R1, R2 formed of groups of apertures 14 form a rhombic lattice, and function as starting lines of deformation to facilitate deformation of the cover nonwoven sheet 20 and the top sheet 30 in the front-back and the width directions.

The deformation of the cover nonwoven sheet 20 and the top sheet 30 in the front-back and the width directions starting from the imaginary linking lines R1, R2, is followed by the easily deformable, liquid-impervious sheet 11 to cause deformation of the latter.

The waist region, which is provided with an elastic member elastically stretchable in the width direction, may prevent body fluid from leaking therethrough.

The elastic member may be selected from various forms and may be, for example, the dorsal stretchable sheet 70 discussed above.

As the elastic stretching and contracting of the dorsal stretchable sheet 70 is followed by the cover nonwoven sheet 20, the top sheet 30, and the liquid-impervious sheet 11 to elastically stretch and contract in the width direction, the Moroccan pattern D shown in Fig. 7 is prone to deformation along the imaginary linking lines R1, R2, but is not prone to deformation in non-perforated regions Z each encircled by the imaginary lines 14q connecting the adjacent apertures 14.

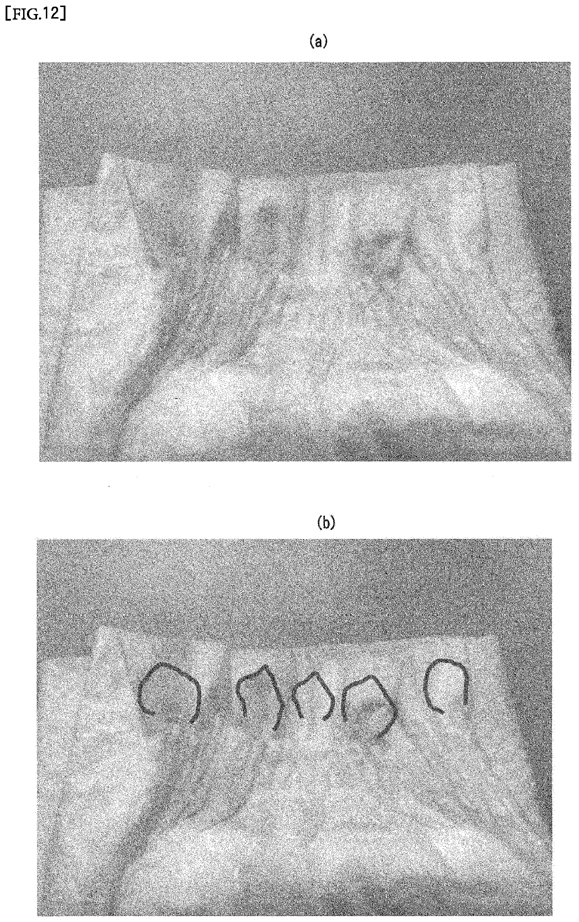

As a result, in elastic stretching and contracting in the width direction, larger ridges are formed on the cover nonwoven sheet 20 side, and larger frills are formed in the dorsal end portion beyond the dorsal stretchable sheet 70, as shown in Fig. 12.

Larger ridges and larger frills represent higher air permeability from inside to outside the diaper, which avoids dampness and skin irritation.

In the embodiment, the Moroccan pattern is formed all over the cover nonwoven sheet 20 and the top sheet 30 in the front-back direction. Formation of the Moroccan pattern D may only be in an end portion, for example, in the dorsal end portion, of a disposable diaper, but in view of difficulties in positional control of the aperture formation in the cover nonwoven fabric and the top sheet material in the production process, and for securing air-permeability along the entire length, it is preferred to form the Moroccan pattern D all over the cover nonwoven sheet 20 and the top sheet 30 in the front-back direction.

In this light, in the embodiment, the Moroccan pattern D is formed in the ventral end flap EF and the dorsal end flap EF, where the absorber body 56 having a certain rigidity is not present.

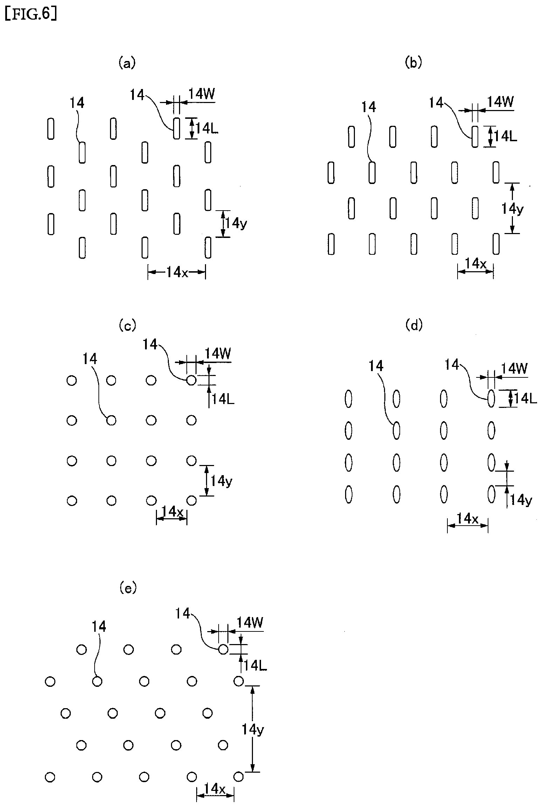

The opening shape of each aperture in the Moroccan pattern D may be elongate as shown in Figs. 6(a) and 6(b), perfect circular as shown in Figs. 6(c) and 6(e), elliptical as shown in Fig. 6(d), polygonal, such as triangular, rectangular, or rhombic, start shaped, cloud shaped, or any arbitrary shape.

The size of each aperture 14 in the Moroccan pattern D is not particularly limited and, referring to Fig. 6, the maximum dimension 14L in the front-back direction LD is preferably 0.3 to 1.8 mm, particularly 0.4 to 1.0 mm, and the maximum dimension 14W in the width direction WD is preferably 0.2 to 1.5 mm, particularly 0.3 to 1.0 mm. When the shape of each aperture 14 is longer in one direction (the overall dimension in one direction is longer than the overall dimension in the direction orthogonal to that direction), such as a slot, elliptical, rectangular, or rhombic shape, the maximum longitudinal dimension is preferably 1.2 to 2.5 times the maximum dimension in the direction orthogonal thereto. Further, when the shape of each aperture 14 is longer in one direction, the longitudinal direction of the apertures 14 is preferably aligned to the front-back direction LD, but may be aligned to the width direction WD or oblique.

The area of each aperture 14 and the area ratio of the apertures 14 may suitably be decided, and the area may preferably be about 0.1 to 2.7 mm² (particularly 0.1 to 1.0 mm²) and the area ratio may preferably be about 1.0 to 15.0 % (particularly 5.0 to 10.0 %).

The opening shape of each aperture in the Moroccan pattern may also be selected from various shapes, such as a circular shape with a diameter of 0.5 to 1.5 mm, or a shape in 0.5 to 1.5 mm size in terms of equivalent diameter, and it is preferred for forming larger ridges and frills that the center-to-center distance between the adjacent apertures is 1.0 to 4.0 mm, and the area of one non-perforated region Z encircled by the imaginary line 14q connecting the apertures in the Moroccan pattern is 1.0 to 3.5 cm².

Note that the dorsal stretchable sheet 70 may be replaced with elastic members, for example, rubber threads, arranged in parallel, without the base sheet 71.

Next, explanations will be made on the components of the embodiment.

### <Cover nonwoven sheet>

Supplemental discussion of the cover nonwoven sheet is being made. The majority of disposable diapers and sanitary napkins are known to have structures in which an air-permeable liquid-impervious sheet is provided on the underside of an absorber body for preventing bleed-through of absorbed liquid while securing air permeability, and this liquid-impervious sheet is covered on its underside with a cover nonwoven sheet for providing fabric-like appearance and texture.

The cover nonwoven sheet 20 in the embodiment, too, is provided for giving fabric-like appearance and texture. The cover nonwoven sheet 20 covers the liquid-impervious sheet 11 on its underside, and constitutes the product external surface in at least part of the region covering the liquid-impervious sheet 11.

In this case, when an air-permeable liquid-impervious sheet is overlaid with a cover nonwoven sheet, the air-permeability is lowered by the presence of the cover nonwoven sheet. One preferred technique for solving this problem is to employ, as a cover nonwoven sheet, perforated nonwoven fabric having a number of apertures penetrating the two sides of the fabric.

In the illustrated embodiment, the cover nonwoven sheet 20 is formed of perforated nonwoven fabric having at intervals a number of apertures 14 penetrating the two sides of the fabric. The kind of fibers or the processing method in fiber bonding (interlacing) of the cover nonwoven sheet 20 is not particularly limited, and may suitably be selected. It is preferred to use an air-through nonwoven fabric with a preferred basis weight of 20 to 30 gsm, and a preferred thickness of 0.3 to 1.0 mm.

The cover nonwoven sheet 20 may be provided, in view of improvement in air permeability, with the apertures 14 all over the front-back and the width directions, in case of tape-type disposable diapers.

As discussed above, the plan shape (opening shape) of each aperture 14 may suitably be decided, including the shapes shown in Figs. 6(a) to 6(e).

### (Top sheet)

The top sheet 30 has a property to permeate liquid, and may be of, for example, perforated or non-perforated nonwoven fabric or porous plastic sheet. Among these, the nonwoven fabric is not particularly limited in its raw material fibers. For example, synthetic fibers, such as olefin-based including polyethylene or polypropylene, polyester-based, or polyamide-based fibers, recycled fibers, such as rayon or cupra, natural fibers, such as cotton, or mixed fibers or composite fibers of two or more of these may be used. Further, the nonwoven fabric may have been produced through any processing. The processing may include known processes, such as spunlacing, spunbonding, thermal bonding, melt-blowing, needle punching, air through, and point bonding. For example, when flexibility or draping properties are required, spunbonding or spunlacing is preferred, whereas when bulkiness or softness is required, air through, point bonding, or thermal bonding is preferred.

In particular, nonwoven fabric produced by air through method is preferred in view of bulkiness and softness.

The nonwoven fabric fibers may be, for example, of PE/PET of 1.5 to 3.5 dtex.

The basis weight of the top sheet 30 is preferably 10 to 30 gsm. At less than 10 gsm, back flow of the body liquid may occur, whereas at over 30 gsm, sufficient softness may be hard to obtain.

The top sheet 30 may be made of one sheet, or of a laminated sheet obtained by bonding two or more sheets together. Similarly, the top sheet 30, in the planar direction, may be made of one sheet or of two or more sheets.

The top sheet 30 may be folded around along the side edges of and onto the underside of the absorbent element 50, or may extend beyond the side edges of the absorbent element 50 without being folded.

The top sheet 30 is preferably fixed to the member contiguous on its underside by joining means through material melt-bonding, such as heat sealing or ultrasonic sealing, or with a hot melt adhesive, for the purpose of avoiding displacement with respect to the underside member. In the illustrated embodiment, the top sheet 30 is fixed, with the hot melt adhesive applied on its underside, to the surface of the intermediate sheet 40 and to the surface of an area of the packing sheet 58 located over the top side of the absorber body 56.

### <Plan arrangement of apertures in top sheet>

The top sheet 30 is preferably formed of perforated nonwoven fabric having the apertures 14 in order to facilitate prompt passage of body fluid to the absorbent element 50. The plan arrangement of the apertures 14 may be in a regularly repeated pattern, such as a rhombic lattice pattern as shown in Fig. 6(a), a hexagonal lattice pattern as shown in Fig. 6(b) (also referred to as a staggered pattern), a square lattice pattern as shown in Fig. 6(c), a rectangular lattice pattern as shown in Fig. 6(d), a parallelogrammatic lattice pattern as shown in Fig. 6(e) (as illustrated, a pattern having two intersecting groups of a number of slanted parallel lines), or the like pattern (including those slanted by less than 90 degrees with respect to the front-back direction LD), as well as a pattern wherein groups of apertures 14 (the arrangement in each unit group may be regular or irregular, and may be in a pattern, letter, or the like) are repeated regularly.

The distance 14y in the front-back direction and the distance 14x in the width direction between adjacent apertures 14 in the top sheet may suitably be decided and, in view of air-permeability, preferably 14y may be 0.9 to 8.0 mm and 14x may be 2.0 to 10 mm, in particular, 14y is 1.0 to 3.0 mm and 14x is 3.0 to 5.0 mm. In particular, as shown in Fig. 6(d), it is preferred that lines of apertures 14 arranged in the front-back direction at front-back intervals 14y smaller than the front-back dimension 14L of each aperture 14 are repeated at predetermined intervals in the width direction WD, with the interval 14x in the width direction being larger than the front-back dimension 14L of each aperture 14 (more preferably, three times or more the dimension 14W of each aperture 14 in the width direction), which leads to significant increase in air permeability without losing softness and bulkiness, and without decrease in tensile strength of the sheet in the front-back direction, which is important during production, and thus is preferred. In this case, it is particularly preferred that the shape of each aperture 14 is elongate in the front-back direction LD.

Of course, in bonding the top sheet 30 and the intermediate sheet 40, thermal adhesion or ultrasonic adhesion may be used, but hot melt adhesion is preferred for securing softness.

### (Intermediate sheet)

For promptly passing the liquid penetrating the top sheet 30 to the absorber body, an intermediate sheet (also referred to as a second sheet) 40 may be provided, of which liquid permeation rate is faster than that of the top sheet 30. This intermediate sheet 40 is capable not only of promptly passing liquid to the absorber body to increase absorption performance by the absorber body, but also preventing "back flow" phenomenon of the absorbed liquid back from the absorber body to keep the top sheet 30 surface always dry. The intermediate sheet 40 may be omitted.

The intermediate sheet 40 may be of the materials similar to those for the top sheet 30, or spunlaced, spunbonded, SMS, or pulp nonwoven fabric, pulp-rayon composite sheets, point-bonded fabric, or crepe paper. In particular, air-through nonwoven fabric is preferred for its bulkiness. For air-through nonwoven fabric, composite fibers of core-shell structure are preferably used, wherein the resin for the core may be polypropylene (PP), or preferably polyester (PET), which is highly rigid. The basis weight is preferably 20 to 80 gsm, more preferably 25 to 60 gsm. The fineness of the raw material fibers of the nonwoven fabric is preferably 2.0 to 10 dtex. For making nonwoven fabric bulky, it is also preferred to use eccentric fibers having off-centered cores, hollow fibers, or eccentric hollow fibers, entirely as the raw material fibers or partially mixed fibers.

In the illustrated embodiment, the width of the intermediate sheet 40 is shorter than the width of the absorber body 56 and arranged in center, but the intermediate sheet 40 is may be provided over the entire width. The longitudinal dimension of the intermediate sheet 40 may be the same as the entire length of the diaper, the same as the length of the absorbent element 50, or within a short length around the liquid receiving area.

The intermediate sheet 40 is preferably fixed to the member contiguous on its underside by joining means through material melt-bonding, such as heat sealing or ultrasonic sealing, or with a hot melt adhesive, for the purpose of avoiding displacement with respect to the underside member. In the illustrated embodiment, the intermediate sheet 40 is fixed, with the hot melt adhesive applied on its underside, to the surface of that area of the packing sheet 58 which is located over the top side of the absorber body 56.

### (Liquid impervious sheet)

Materials of the liquid-impervious sheet 11 is not particularly limited, and may be, for example, plastic film made of olefin-based resins or the like, such as polyethylene or polypropylene, laminated nonwoven fabric wherein plastic film is laminated over nonwoven fabric, or a laminated sheet wherein nonwoven fabric or the like is laid over and joined on plastic film. The liquid-impervious sheet 11 may be made of a material which is preferably used for preventing dampness and is not liquid-pervious and is moisture-permeable. As moisture-permeable plastic film, microporous plastic film is widely used, which is obtained by kneading an inorganic filler in an olefin-based resin, such as polyethylene or polypropylene, molding the resulting mixture into a sheet, and then uni- or biaxially drawing the sheet. Also, nonwoven fabric of microdenier fibers, or sheets that have been rendered liquid-impervious without using plastic film through a process, such as enhancement of leak proof property by applying heat or pressure to minimize interfiber gaps, or coating with a highly water-absorbable resin or a hydrophobic resin or water repellent, may be used as the liquid-impervious sheet 11. For sufficient bonding strength in bonding to a cover nonwoven sheet 20 via a hot melt adhesive as will be discussed later, use of plastic film is preferred.

The liquid-impervious sheet 11 may have a width to fit behind the absorbent element 50 as illustrated, or may be folded around each side edge of the absorbent element 50 to extend to the corresponding side of the top sheet 30. The width of such extensions may suitably be about 5 to 20 mm on each opposed side.

Inside of the liquid-impervious sheet 11, in particular on the side faces of the absorber body 56, an excretion indicator which changes in color upon absorption of a liquid component, may be provided.

### (Side gather part)

Each side gather part 60 extends all over the front-back direction LD, and is provided to be brought into contact around each leg of a wearer to prevent side leakage. Those generally referred to as three-dimensional gathers 69 and planar gathers 61 fall under this part.

The gather nonwoven fabric 62 may preferably be flexible nonwoven fabric having excellent uniformity and concealability, such as spunbonded nonwoven fabric (SS, SSS, or the like), SMS nonwoven fabric (SMS, SSMMS, or the like), or melt-blown nonwoven fabric, which may have been subjected to water-repellent treatment with silicone or the like, as required. The basis weight of the fibers may preferably be about 10 to 30 gsm. The elongate elastic members 63 may be of rubber thread or the like. When spandex rubber thread is used, the fineness is preferably 470 to 1240 dtex, more preferably 620 to 940 dtex. The stretch rate in the fixed state is preferably 150 to 350 %, more preferably 200 to 300 %. Note that the term "stretch rate" refers to a value with respect to the natural length being 100 %. Further, as illustrated, a waterproof film may be interposed between duplicate gather nonwoven fabric 62. In this case, the gather nonwoven fabric 62 may partially be omitted in the area where the waterproof film is present, but in order to impart fabric-like appearance and texture to the product, the exterior of each side gather part 60 at least from its root end to its leading edge is required to be formed of the gather nonwoven fabric 62, as in the illustrated embodiment.

The number of the elongate elastic members 63 provided in the free section of each side gather part 60 is preferably 2 to 6, more preferably 3 to 5.

### (Absorbent element)

The absorbent element 50 includes the absorber body 56 and the packing sheet 58 packing the entire absorber body 56.

### (Absorber body)

The absorber body 56 may be formed of an assembly of fibers. Such an assembly of fibers may be an accumulation of short fibers of fluff pulp, synthetic fibers, or the like, as well as an assembly of filaments obtained by opening, where necessary, a tow (fiber bundle) of synthetic fibers, such as cellulose acetate. The basis weight of the fibers may be about 100 to 300 gsm for an accumulation of fluff pulp or short fibers, and about 30 to 120 gsm for an assembly of filaments. The fineness of the synthetic fibers, when used, is, for example, 1 to 16 dtex, preferably 1 to 10 dtex, more preferably 1 to 5 dtex. In an assembly of filaments, the filaments may be of uncrimped fibers, but crimped fibers are preferred. The number of crimps of the crimped fibers may be, for example, 5 to 75, preferably 10 to 50, more preferably 15 to 50 per inch. Uniformly crimped fibers are often used. In the absorber body 56, superabsorbent polymer particles are preferably dispersed and retained.

The absorber body 56 may be in a rectangular shape, or in the shape of an hourglass having the front end, the back end, and a narrowed section located between the front and back ends and having a narrower width compared to the front and back ends.

The size of the absorber body 56 may suitably be decided as long as the absorber body extends to the front, back, left, and right of the position of the urination port.

### (Superabsorbent polymer particles)

The absorber body 56 may be caused partially or entirely to contain superabsorbent polymer particles. The superabsorbent polymer particles include not only "particles", but also "powders". The superabsorbent polymer particles may be those used in this type of disposable diapers as they are, and may preferably be particles 30 wt% or less of which, after sieving (five-minute shaking), remain on a 500 µm standard sieve (JIS Z8801-1: 2006) and particles 60 wt% or more of which, after sieving (five-minute shaking), remain on a 180 µm standard sieve (JIS Z8801-1: 2006).

Any material of the superabsorbent polymer particles may be used without particular limitation, and those having a water absorption of 40 g/g or more are preferred. The superabsorbent polymer particles may be of starch-based, cellulose-based, or synthetic polymer-based. Starch-acrylic acid (salt) graft copolymers, saponified products of starch-acrylonitrile copolymers, cross-linked sodium carboxymethyl cellulose, or acrylic acid (salt) polymers may be used. The superabsorbent polymer particles may preferably be in ordinary powder or granular form, but particles in other forms may also be used.

The superabsorbent polymer particles having a water absorption rate of 70 seconds or less, particularly 40 seconds or less, may preferably be used. With too slow a water absorption rate, so-called back flow may likely to occur, wherein liquid supplied into the absorber body 56 returns out of the absorber body 56.

The superabsorbent polymer particles may preferably be those having a gel strength of 1000 Pa or higher. With such property, when the superabsorbent polymer particles are formed into a bulky absorber body 56, stickiness after liquid absorption may effectively be limited.

The basis weight of the superabsorbent polymer particles may suitably be decided depending on the absorption amount required in a use of the absorber body 56. Thus, it depends, but the basis weight may be 50 to 350 gsm. At a basis weight of the polymer less than 50 gsm, the absorption amount may hardly be secured. At over 350 gsm, the effect may be saturated.

Where necessary, the spread density or spread amount of the superabsorbent polymer particles may be adjusted in the horizontal direction of the absorber body 56. For example, the spread amount on the liquid excretion area may be larger than that on the remaining areas. Considering the sexual difference, the spread density (amount) on the front side may be higher for men's, whereas the spread density (amount) in the center portion may be higher for women's. Further, the absorber body 56 in its horizontal direction may be provided with a local (e.g., spot) area without the polymer.

### (Packing sheet)

For limiting escape of the superabsorbent polymer particles, or for improving maintenance of the shape of the absorber body 56, the absorber body 56 is wrapped with a packing sheet 58.

The material of the packing sheet 58, when used, may be tissues, in particular, crepe paper, nonwoven fabric, polyethylene-laminated nonwoven fabric, perforated sheet, or the like.

Conventionally, crepe paper is often used. In the present invention, SMS nonwoven fabric (spunbonded/melt-blown/spunbonded laminated nonwoven fabric) or SMMS nonwoven fabric (spunbonded/melt-blown/melt-blown/spunbonded laminated nonwoven fabric) is used.

With crepe paper, in which pulp fibers extend in the front-back direction (MD) and arranged densely, rigidity particularly in the front-back direction is high (with less softness).

In contrast, with SMS or SMMS nonwoven fabric, for example, rigidity particularly in the front-back direction is low (with excellent softness), and bending rigidity in the front-back direction (MD) and in the 45-degree oblique direction is lower, compared to those with crepe paper, as will be shown by the results of cantilever test.

As a result, the disposable diaper, when worn, deforms well (bends well) in response to the posture change of the wearer and is securely kept in contact with the skin of the wearer, which leads to improved leak prevention.

The materials of the SMS nonwoven fabric or SMMS nonwoven fabric may be polypropylene, polyethylene/polypropylene composite material, or the like. In particular, nonwoven fabric subjected to hydrophilization treatment for improving body fluid absorption characteristics is preferred.

The materials having a basis weight of preferably 5 to 40 gsm, particularly 10 to 30 gsm are preferred.

How to pack with the packing sheet 58 may suitably be decided and, in view of readiness of production or protection against leakage of the superabsorbent polymer particles through the front or back end edge, preferably the packing sheet 58 is wrapped cylindrically around the absorber body 56 to surround its top and under faces as well as both side faces, with the front and back edge portions of the packing sheet extending forwardly and backwardly beyond the absorber body 56, and the overlaid wrapping portion as well as the overlapped portions in the front and back extensions are joined with joining means, such as a hot melt adhesive or material melt-bonding.

Where needed, the absorber body 56 may be covered only on its top and under faces with two separate sheets of nonwoven fabric, with both side faces being uncovered.

### <Explanation of terms in the specification>

The following terms appearing in the present specification shall have the following meaning unless otherwise specified herein.

- The "front-back (longitudinal) direction" refers to the direction connecting the ventral side (front side) and the dorsal side (back side), whereas the "width direction" refers to the direction orthogonal to the front-back direction (right-left direction).
- The "top side" refers to the side of a tape-type disposable diaper, when worn, closer to the skin of the wearer, whereas the "underside" refers to the side of a tape-type disposable diaper, when worn, away from the skin of the wearer.
- The "top face" refers to the face of a tape-type disposable diaper, when worn, closer to the skin of the wearer, whereas the "under face" refers to the face of a tape-type disposable diaper, when worn, away from the skin of the wearer.
- The "area ratio" refers to the ratio of the objective area per unit area, and is represented in percentage by dividing the sum of the areas of objective portions (e.g., apertures) in an objective region (e.g., cover nonwoven sheet) by the area of that objective region. In a configuration where a number of objective portions are provided at intervals, the area ratio is preferably determined with the objective region being set to a size containing 10 or more objective portions. For example, the area ratio of the apertures may be determined in the following procedure, using, for example, VHX-1000 (tradename) manufactured by KEYENCE under the measurement conditions in x200 magnification.
   (1) Place a specimen under a ×20 magnification lens, and adjust the focus. Position the nonwoven fabric so that 4 × 6 apertures are in the field.
   (2) Specify the brightness of the aperture portions, and measure the area of the apertures.
   (3) Click the color extraction in "Area Measurement" under "Measurement and Comment". Click the aperture portions.
   (4) Click "Collective Measurement", check "Display the measurement result window", and store in CSV data.
- The "stretch rate" refers to a value with respect to the natural length being 100 %.
- The "gel strength" is determined as follows. To 49.0 g of artificial urine (a mixture of 2 wt% urea, 0.8 wt% sodium chloride, 0.03 wt% calcium chloride dihydrate, 0.08 wt% magnesium sulfate heptahydrate, and 97.09 wt% ion-exchanged water), 1.0 g of superabsorbent polymer is added and stirred with a stirrer. The resulting gel is left in a chamber with constant temperature and humidity at 40 °C at 60 % RH for 3 hours, and then the temperature is returned to the ordinary temperature. The gel strength is measured in a curd meter (Curdmeter-MAX ME-500 manufactured by I. techno Engineering).
- The "basis weight" is determined as follows. A specimen or test piece is preliminarily dried, left in a laboratory or in apparatus under the standard conditions (20 ± 5 °C temperature and 65 % or lower relative humidity in the testing location) until constant mass is attained. The preliminary drying means attaining constant mass from a specimen or test piece in the environment not exceeding a relative humidity of 10 to 25 % and a temperature of 50 °C. No preliminary drying may be performed on fibers with an official regain of 0.0 %. From the test piece of the constant mass, a specimen of 200 mm × 250 mm size (± 2 mm) is cut out, using a plate of 1 gsm (200 mm × 250 mm, ± 2 mm). The weight of the specimen is measured and multiplied by 20 times to calculate the weight per 1 m², which is taken as the basis weight.
- The "thickness" is automatically measured using an automatic thickness meter (KES-G5 handy compression tester program) under a load of 10 gf/cm² with the compression area of 2 cm².
- The water absorption is determined in accordance with JIS K7223 - 1996 "Testing method for water absorption capacity of super absorbent polymers".
- The water absorption rate is defined as the "time spent until the end point is reached" in carrying out JIS K7224 - 1996 "Testing method for water absorption rate of super absorbent polymers" using 2 g of superabsorbent polymer and 50 g of saline.
- The "spread state" refers to the state in which an article is unfolded and laid flat without contraction or slack.
- The size of each part refers to the size not in the natural length state but in the spread state, unless otherwise specified.
- Unless the environmental conditions of a test or measurement are otherwise specified, the test or measurement shall be conducted in a laboratory or in apparatus under the standard conditions (20 ± 5 °C temperature and 65 % or lower relative humidity in the testing location).

Next, Examples and Comparative Examples are disclosed to demonstrate the effects of the present invention.

### <Prototype>

### (Example)

A tape-type disposable diaper of a structure as shown in Figs. 1 to 5 was produced using, as the cover nonwoven sheet 20, air-through nonwoven fabric which had been prepared by air-through method from fiber material of PE/PET = 50/50 in 2.5 dtex, and had a basis weight of 20 gsm. The cover nonwoven sheet 20 had apertures arranged in the Moroccan pattern of which dimensions as shown in Fig. 7 are as follows:
Diameter of circular aperture 14: 1.0 mm
14px, 14py = 2.5 mm
14X = 16mm
14Y = 18mm

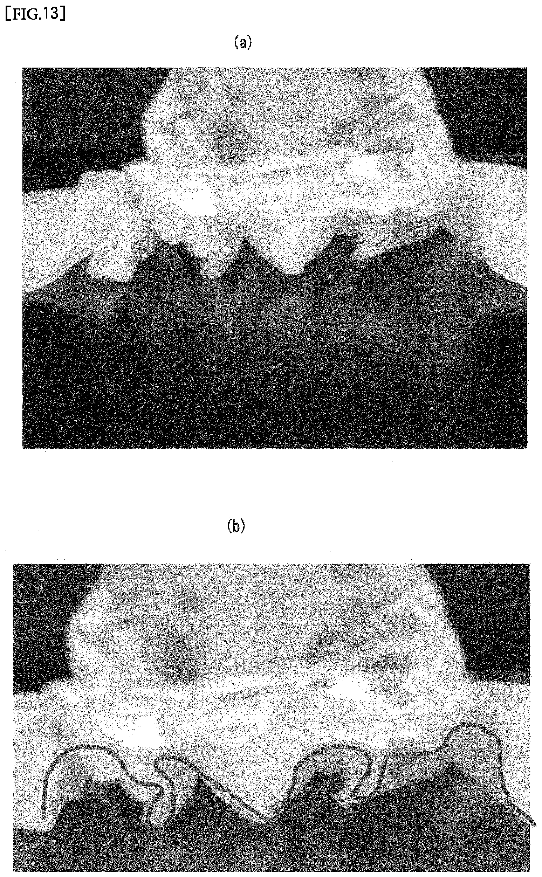

On the dorsal cover sheet side of the obtained tape-type disposable diaper, large ridges were formed as shown in Fig. 12 and large fills were formed as shown in Fig. 13.

Note that Figs. 12(b) and 13(b) are photographs partially processed by the applicant for manifesting the ridges and frills in the photographs of Figs. 12(a) and 13(a), respectively.

### (Comparative Example 1)

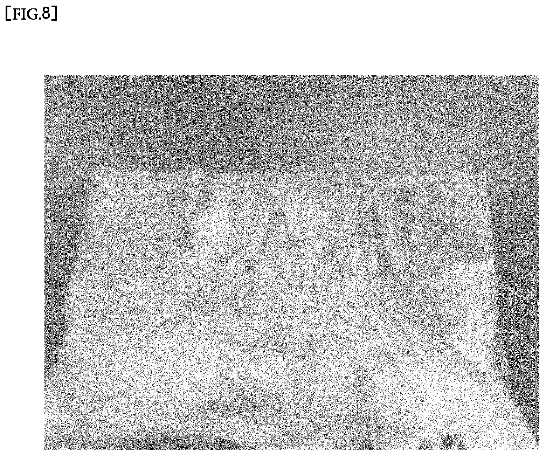

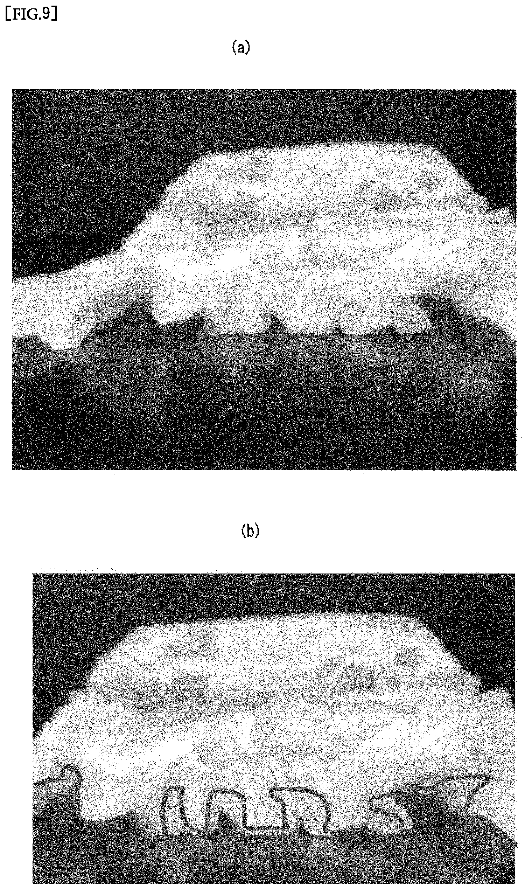

A tape-type disposable diaper was obtained in the same way, except that the apertures were not formed, to have, on its dorsal cover sheet side, no large ridges as shown in Fig. 8 and no large frills as shown in Fig. 9.

### (Comparative Example 2)

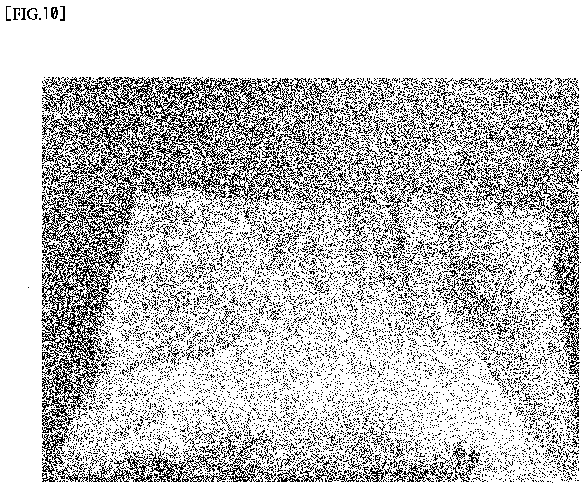

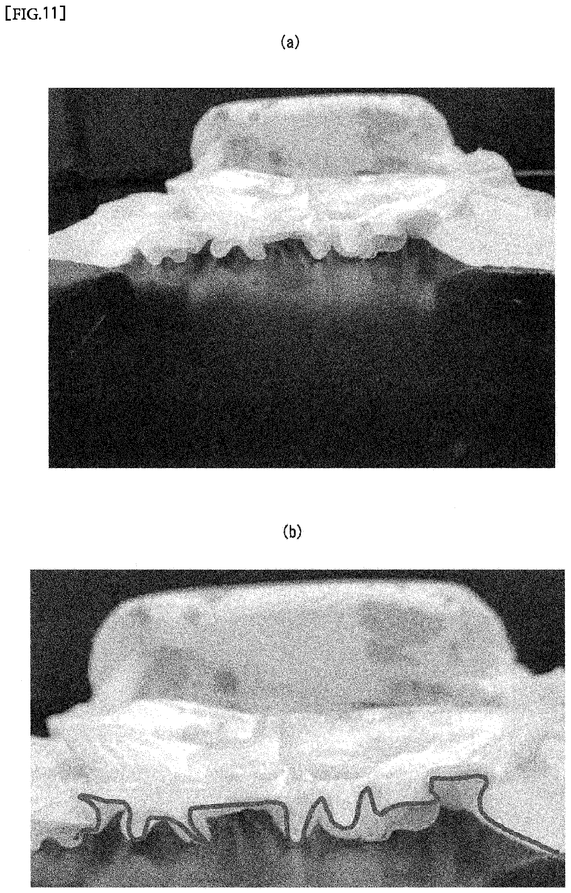

A disposable diaper was obtained in the same way, except that the apertures were arranged in the staggered pattern as shown in Fig. 6(e), to have, on its dorsal cover sheet side, no large ridges as shown in Fig. 10 and no large frills as shown in Fig. 11. The diameter of each circular aperture 14 was 1.0 mm.

According to the Example, in comparison with Comparative Examples 1 and 2, larger ridges and larger frills were formed, which revealed that the diaper, when worn, provided excellent air permeability and were hard to cause skin rash.

### INDUSTRIAL APPLICABILITY

The present invention may be applied to disposable diapers in general, including not only tape-type disposable diapers, but also pad-type disposable diapers and underpants-type disposable diapers.

### DESCRIPTION OF REFERENCE NUMERALS

- 11: liquid-impervious sheet
- 20: cover nonwoven sheet
- 20H: hot melt adhesive
- 14: aperture
- 30: top sheet
- 40: intermediate sheet
- 50: absorbent element
- 56: absorber body
- 58: packing sheet
- 60: side gather part
- 62: gather nonwoven fabric
- LD: front-back direction
- WD: width direction

## Claims

1. A disposable diaper comprising a top sheet constituting a use side surface, a liquid-impervious sheet provided on an under face side, and an absorbent element interposed therebetween,
wherein a cover nonwoven sheet is provided on an under face side of the liquid-impervious sheet, the cover nonwoven sheet being formed of perforated nonwoven fabric having a number of apertures arranged at intervals and each penetrating two sides of the fabric, and
imaginary lines connecting the adjacent apertures located in a waist region of the cover nonwoven sheet form a Moroccan pattern, and at least part of the Moroccan pattern does not overlap an absorber body contained in the absorbent element.

2. The disposable diaper according to claim 1, wherein the waist region is provided with an elastic member which is elastically stretchable in a width direction.

3. The disposable diaper according to claim 1, wherein each aperture is in a circular form with a 0.5 to 1.5 mm diameter, a center-to-center distance between adjacent ones of the apertures is 1.0 to 4.0 mm, and an area of one non-perforated region encircled by the imaginary lines connecting the apertures in the Moroccan pattern is 1.0 to 3.5 cm².

4. The disposable diaper according to claim 1, wherein the cover nonwoven sheet has a basis weight of 10 to 30 gsm.
